# EUROPÄISCHE PATENTSCHRIFT

(11) **EP 3 878 395 B1**
(45) Veröffentlichungstag und Bekanntmachung des Hinweises auf die Patenterteilung: **26.04.2023**
(21) Anmeldenummer: 21162195.8
(22) Anmeldetag: 12.03.2021
(51) Int. Cl.: A61B 50/30, A61B 50/00

(54) **MEDIZINISCHER VERPACKUNGSEINSATZ, MEDIZINISCHES VERPACKUNGSSYSTEM UND VERFAHREN ZUM VERPACKEN EINES MEDIZINISCHEN OBJEKTS**
MEDICAL PACKAGING INSERT, MEDICAL PACKAGING SYSTEM AND METHOD FOR PACKAGING A MEDICAL OBJECT
INSERT D'EMBALLAGE MÉDICAL, SYSTÈME D'EMBALLAGE MÉDICAL ET PROCÉDÉ D'EMBALLAGE D'UN OBJET MÉDICAL

(30) Priorität: 13.03.2020 DE 102020106955
(43) Veröffentlichungstag der Anmeldung: 15.09.2021
(73) Patentinhaber: Aesculap AG, 78532 Tuttlingen (DE)
(72) Erfinder: Sauter, Wolfgang, 78603 Renquishausen (DE); Kammerer, Selina, 78083 Dauchingen (DE); Juppenlatz, Domenic, 78532 Tuttlingen (DE)
(74) Vertreter: Hoeger, Stellrecht & Partner Patentanwälte mbB

(56) Entgegenhaltungen:
- EP-A2- 1 069 056
- US-A- 5 816 403
- US-A1- 2011 240 515
- US-A1- 2019 274 809

## Beschreibung

Die vorliegende Erfindung betrifft einen medizinischen Verpackungseinsatz für ein medizinisches Verpackungssystem zum Aufnehmen mindestens eines medizinischen Objekts, wobei der Verpackungseinsatz eine Behälterwanne umfasst, die einen Aufnahmeraum definiert, und durch thermisches Umformen aus einem ebenen, eine Schichtmaterialebene definierenden Schichtmaterial ausgebildet ist, wobei an der Behälterwanne zwei voneinander weg weisende Laschen ausgebildet sind, die in einer Grundstellung quer, insbesondere senkrecht, von aufeinander zu weisenden Wandflächen der Behälterwanne abstehen.

Ferner betrifft die vorliegende Erfindung ein medizinisches Verpackungssystem zum, insbesondere sterilen, Verpacken mindestens eines medizinischen Objekts, wobei das Verpackungssystem einen ersten Verpackungsbehälter umfasst, welcher einen ersten Verpackungsbehälteraufnahmeraum definiert zum Aufnehmen mindestens eines medizinisches Verpackungseinsatzes und des mindestens einen medizinischen Objekts, das im medizinischen Verpackungseinsatz aufgenommen ist.

Des Weiteren betrifft die vorliegende Erfindung ein Verfahren zum Verpacken eines medizinischen Objekts in einem Verpackungseinsatz der eingangs beschriebenen Art.

Medizinische Objekte, insbesondere Implantate, Implantatteile oder chirurgische Instrumente, werden häufig steril verpackt. Hierzu dienen insbesondere Verpackungsbehälter, die einen Verpackungsbehälteraufnahmeraum definieren zum Aufnehmen des Objekts. Der Verpackungsbehälter kann insbesondere aus einer Folie ausgebildet und mit einer Oberfolie verschlossen sein.

Es ist bekannt, das medizinische Objekt in einen Verpackungseinsatz einzubetten, um insbesondere das Objekt und auch den Verpackungsbehälter, der als Sterilverpackung ausgebildet ist, zu schützen. Der Verpackungseinsatz verhindert insbesondere eine Beschädigung der Sterilbarriere der Sterilverpackung und auch einen Partikelabrieb, der durch Relativbewegung zwischen dem medizinischen Objekt und dem Verpackungsbehälter, der wie erwähnt aus einer oder mehreren Folien ausgebildet sein kann, beim Transport der Sterilverpackung mit dem darin enthaltenen Objekt entstehen kann, zu verhindern.

Bekannte Verpackungseinsätze sind wannenförmig ausgebildet und oben offen, sodass das medizinische Objekt auf einfache Weise in diesen eingesetzt werden kann. Nachteil dabei ist jedoch, dass Relativbewegungen zwischen der den Verpackungseinsatz übergreifenden Oberfolie der Sterilverpackung und dem medizinischen Objekt nicht entkoppelt sind, wodurch es im ungünstigsten Fall zu einem unerwünschten Partikelabrieb kommen kann.

Aus der US 2019/0274809 A1 ist eine Implantatverpackungsanordnung bekannt. Eine Packung zum Lagern von Sterilgut, eine Vorrichtung und Verfahren zum Herstellen einer derartigen Packung sind in der EP 1 069 056 A2 beschrieben. Sterilisierbare Behälter für die sterile Darreichung ihres Inhalts sind in der US 5,816,403 offenbart. Die US 2011/0240515 A1 betrifft ein Verpackungssystem.

Es ist daher eine Aufgabe der vorliegenden Erfindung, einen medizinischen Verpackungseinsatz, ein medizinisches Verpackungssystem und ein Verfahren zum Verpacken eines medizinischen Objekts so zu verbessern, dass insbesondere ein Partikelabrieb aufgrund eines Kontakts zwischen dem mindestens einen medizinischen Objekt und einem Verpackungsbehälter, insbesondere einer Sterilverpackung, verhindert wird.

Diese Aufgabe wird bei einem medizinischen Verpackungseinsatz der eingangs beschriebenen Art erfindungsgemäß dadurch gelöst, dass der Verpackungseinsatz umstülpbar ausgebildet ist und durch Umstülpen von der Grundstellung in eine Verpackungsstellung, in der die in der Grundstellung aufeinander zu weisenden Wandflächen voneinander weg weisen und die zwei Laschen aufeinander zu weisen und den Aufnahmeraum mindestens teilweise, insbesondere vollständig, verschließen, überführbar ist.

Die vorgeschlagene Weiterbildung eines bekannten medizinischen Verpackungseinsatzes ermöglicht es insbesondere, mit den beiden an der Behälterwanne angeordneten Laschen die Behälterwanne in der Verpackungsstellung zu verschließen. Auf diese Weise ist das mindestens eine medizinische Objekt, das im Verpackungseinsatz in der Verpackungsstellung aufgenommen ist, vollständig oder im Wesentlichen vollständig vom Verpackungseinsatz umgeben und kann daher nicht mit einem Verpackungsbehälter, in dem der Verpackungseinsatz aufgenommen ist, beispielsweise eine aus einer Unterfolie und einer mit dieser verschweißten Oberfolie gebildeten Sterilverpackung, in Kontakt kommen. So kann ein Partikelabrieb am Verpackungsbehälter, also an der Sterilverpackung, in Folge eines Kontakts mit dem mindestens einen medizinischen Objekt sowie einer Relativbewegung desselben zum Verpackungsbehälter wirksam verhindert werden. Ferner lässt sich ein solcher Verpackungseinsatz insbesondere auch einfach herstellen, beispielsweise durch thermisches Umformen und Herauslösen aus dem thermisch umgeformten Schichtmaterial, beispielsweise durch Schneiden oder Stanzen. Überdies hat ein wie vorgeschlagen ausgebildeter Verpackungseinsatz insbesondere auch den Vorteil, dass die zunächst den Aufnahmeraum frei gebenden Laschen beim Umstülpen, auch als Wenden oder Umschlagen des Verpackungseinsatzes bezeichnet, automatisch ihre Orientierung ändern und in der Verpackungsstellung aufeinander zu weisen, so dass sie den Aufnahmeraum teilweise oder vollständig verschließen, je nachdem, wie lang die Laschen ausgebildet sind.

Vorteilhaft ist es, wenn die Behälterwanne von einer Behälterwannenwand begrenzt ist, wenn die Behälterwannenwand in der Grundstellung des Verpackungseinsatzes eine äußere Behälterwandfläche und eine innere Behälterwandfläche definiert, wenn die innere Behälterwandfläche in der Grundstellung den Aufnahmeraum begrenzt und wenn in der Verpackungsstellung die äußere Behälterwandfläche den Aufnahmeraum begrenzt. Durch das Umstülpen des Verpackungseinsatzes wird die zunächst innere Behälterwandfläche, die in der Grundstellung den Aufnahmeraum begrenzt, in der Verpackungsstellung zur äußeren Behälterwandfläche, die vom Verpackungseinsatz weg weist. Entsprechend gilt dies umgekehrt für die äußere Behälterwandfläche. Mit anderen Worten wird das medizinische Objekt nicht in der Behälterwanne aufgenommen, bei der die innere Behälterwandfläche den Aufnahmeraum begrenzt, sondern bei der die in der Grundstellung äußere Behälterwandfläche den Aufnahmeraum begrenzt. Dies kann dadurch erreicht werden, dass beim oder vor dem Einsetzen des medizinischen Objekts in die Behälterwanne des Verpackungseinsatzes der Verpackungseinsatz wie beschrieben umgestülpt wird.

Auf einfache Weise lässt sich der Verpackungseinsatz ausbilden, wenn die zwei Laschen eben oder im Wesentlichen eben ausgebildet sind und in der Grundstellung und/oder in der Verpackungsstellung eine gemeinsame Laschenebene definieren.

Die Herstellung des Verpackungseinsatzes kann insbesondere dadurch vereinfacht werden, dass die Laschenebene durch die Schichtmaterialebene definiert ist. Die Laschen werden in diesem Fall insbesondere durch Bereiche des Schichtmaterials gebildet, die beim thermischen Umformen nicht in ihrer Form verändert werden.

Um den Aufnahmeraum der Behälterwanne vollständig verschließen zu können, ist es günstig, wenn die zwei Laschen in der Verpackungsstellung einander mindestens teilweise überlappen. Insbesondere können sie sich in der Verpackungsstellung auch vollständig überlappen.

Gemäß einer weiteren bevorzugten Ausführungsform der Erfindung kann vorgesehen sein, dass die Behälterwanne einen umlaufenden Rand aufweist, dass der Rand in der Schichtmaterialebene liegt und dass die zwei Laschen sich vom Rand weg erstrecken. Eine solche Behälterwanne kann insbesondere auf einfache Weise aus einem Schichtmaterial geformt werden durch thermisches Umformen.

Vorteilhaft ist es, wenn der Rand zwei Längsseiten und zwei Querseiten aufweist und wenn die zwei Laschen jeweils an einer Querseite des Rands angeordnet oder ausgebildet sind. Diese Ausgestaltung ermöglicht es insbesondere, die Behälterwanne mit einer rechteckigen oder im Wesentlichen rechteckigen Öffnung zum Einführen des mindestens einen medizinischen Objekts in den Aufnahmeraum auszubilden.

Vorzugsweise weisen die zwei Laschen eine Länge ausgehend von der Behälterwanne weg auf, die mindestens etwa einem halben Abstand zwischen den zwei Querseiten entspricht. Wenn beide Laschen eine solche Länge aufweisen, überlappen sie mindestens teilweise in der Verpackungsstellung und verschlie-βen so den Aufnahmeraum der Behälterwanne vollständig. Ein Kontakt zwischen dem aufgenommenen mindestens einen medizinischen Objekt und einem den Verpackungseinsatz umgebenden Verpackungsbehälter kann so wirksam ausgeschlossen werden.

Vorteilhafterweise entspricht die Länge jeder Lasche maximal etwa einem Abstand zwischen den zwei Querseiten. Diese Ausgestaltung hat insbesondere den Vorteil, dass die Laschen in der Verpackungsstellung dann nicht seitlich über die Behälterwanne vorstehen.

Vorteilhaft ist es, wenn die Längsseiten des Rands definierende Randabschnitte flanschlos ausgebildet sind. Flanschlos in diesem Sinne bedeutet insbesondere, dass kein seitlicher Vorsprung im Bereich des Rands, welcher sich quer zu einer Wandfläche der Behälterwanne in diesem Bereich erstreckt, vorgesehen ist. Die Längsseiten des Rands weisen mit anderen Worten eine Breite auf, welche einer Dicke des Schichtmaterials nach dem thermischen Umformen entspricht.

Günstig ist es, wenn die Längsseiten und die Querseiten des Rands über einen Eckbereich verbunden sind, wenn der Eckbereich einen Abschnitt des Rands bildet und wenn der Eckbereich gekrümmt verläuft. Eine solche Ausgestaltung hat insbesondere den Vorteil, dass beim beschriebenen Umstülpen des Verpackungseinsatzes, also insbesondere beim Umstülpen der Behälterwanne, über die Eckbereiche eine hinreichende Zugkraft auf die Laschen ausgeübt werden kann, um diese zum automatischen Umklappen von der Grundstellung, in der sie voneinander weg weisen, in die Verpackungsstellung, in der sie aufeinander zu weisen, zu bringen. Insbesondere kann ein derart geformter Eckbereich einen Winkel zwischen der jeweiligen Lasche und einem Wandbereich der Behälterwanne, der an die Querseiten angrenzt, beim Umstülpen oder Wenden und insbesondere dann auch in der Verpackungsstellung stabilisieren.

Günstigerweise erstreckt sich der Eckbereich in der Schichtmaterialebene und über einen Umfangswinkel in einem Bereich von etwa 85° bis etwa 95°. Insbesondere beträgt der Umfangswinkel etwa 90°. Ein solcher Eckbereich lässt sich auf einfache Weise herstellen. Ferner kann er insbesondere rechtwinklig oder im Wesentlichen rechtwinklig zueinander verlaufende Längs- und Querseiten in gewünschter Weise miteinander verbinden.

Vorteilhaft ist es, wenn ein Krümmungsradius des Eckbereichs einen Wert in einem Bereich von etwa 3 mm bis etwa 20 mm aufweist. Insbesondere kann der Krümmungsradius in einem Bereich von etwa 4 mm bis etwa 8 mm liegen. Derartige Bereiche von Krümmungsradien ermöglichen es insbesondere, die zwei Laschen in der Verpackungsstellung den Aufnahmeraum verschließend zu halten.

Einfach ausbilden lässt sich der medizinische Verpackungseinsatz, wenn sich die Querseiten des Rands zwischen zwei Eckbereichen geradlinig erstrecken.

Günstig ist es, wenn sich die Längsseiten des Rands zwischen zwei Eckbereichen geradlinig oder im Wesentlichen geradlinig erstrecken oder wenn sich die Längsseiten des Rands zwischen zwei Eckbereichen konvex vom Verpackungseinsatz weg weisend erstrecken. Die Längsseiten in der beschriebenen Weise auszubilden ist auf einfache Weise möglich. Insbesondere geradlinig verlaufende Längsseiten vorzusehen ermöglichen es, medizinische Verpackungseinsätze aus einem Schichtmaterial mit minimalem Verschnitt auszubilden.

Gemäß einer weiteren bevorzugten Ausführungsform der Erfindung kann vorgesehen sein, dass Übergangsbereiche zwischen der Behälterwanne und den zwei Laschen eine Steifigkeit aufweisen derart, dass ein zwischen der Behälterwanne und der jeweiligen Lasche in den Übergangsbereichen eingeschlossener Flanschwinkel nach dem Überführen des Verpackungseinsatzes von der Grundstellung in die Verpackungsstellung erhalten bleibt oder im Wesentlichen erhalten bleibt. Die Übergangsbereiche in der beschriebenen Weise auszubilden hat insbesondere den Vorteil, dass die Laschen in der Grundstellung in definierter Weise voneinander weg weisen und beim Umstülpen des Verpackungseinsatzes automatisch aufeinander zu weisen. Eine solche Steifigkeit kann insbesondere auf unterschiedliche Arten erreicht werden. Dies kann insbesondere durch eine besondere Formgebung erreicht werden, wie beispielsweise gekrümmte Eckbereiche wie oben beschrieben, oder durch eine geeignete Materialwahl, aus der der Verpackungseinsatz ausgebildet ist. Zudem kann eine Steifigkeit auch insbesondere durch das thermische Umformen eingestellt werden, also beispielsweise durch gezielte Verfestigung des Schichtmaterials in den Übergangsbereichen.

Vorzugsweise weist der Flanschwinkel einen Wert in einem Bereich von etwa 85° bis etwa 95° auf. Insbesondere kann er etwa 90° betragen. Mit einem derartigen Flanschwinkel können die Laschen in der Grundstellung voneinander weg weisen und in der Verpackungsstellung aufeinander zu.

Um eine möglichst gute Bedeckung des Aufnahmeraums mit den zwei Laschen in der Verpackungsstellung sicherstellen zu können, ist es vorteilhaft, wenn eine Differenz des Flanschwinkels in der Grundstellung und des Flanschwinkels in der Verpackungsstellung nicht größer als 30° ist. Insbesondere ist es vorteilhaft, wenn die Differenz nicht größer als 15° ist. Beträgt beispielsweise der Flanschwinkel in der Grundstellung 90°, dann sollte der Flanschwinkel in der Verpackungsstellung nicht kleiner als 60° oder größer als 120° sein, insbesondere nicht kleiner als 75° oder größer als 105°.

Die Handhabung des Verpackungseinsatzes wird besonders einfach, wenn der Verpackungseinsatz einstückig, insbesondere monolithisch, ausgebildet ist. Zudem lässt er sich so in einem einzigen thermischen Umformschritt ausbilden.

Vorzugsweise liegt eine Wandstärke des Verpackungseinsatzes in einem Bereich von etwa 0,05 mm bis etwa 10 mm. Eine Wandstärke des Verpackungseinsatzes hängt insbesondere vom gewählten Material ab, aus dem der Verpackungseinsatz ausgebildet ist. Die Wandstärke wird abhängig vom Material insbesondere so gewählt, dass sich der Verpackungseinsatz noch in der vorgeschlagenen Weise umstülpen oder umschlagen lässt.

Günstigerweise entspricht die Wandstärke einer Dicke des Schichtmaterials oder im Wesentlichen der Dicke des Schichtmaterials. Mit anderen Worten wird beim thermischen Umformen die Dicke des Schichtmaterials nicht oder nur unwesentlich geändert.

Der Verpackungseinsatz lässt sich insbesondere auf einfache Weise herstellen, wenn er aus einem Kunststoff ausgebildet ist. Insbesondere kann es sich dabei um thermoplastischen Kunststoff handeln. Ein thermoplastischer Kunststoff lässt sich auf einfache Weise thermisch umformen.

Vorzugsweise ist der Kunststoff geschäumt oder extrudiert oder in Form eines Vliesstoffs ausgebildet. Die vorgeschlagenen Ausgestaltungen ermöglichen es insbesondere, mindestens ein medizinisches Objekt, insbesondere einziges, gut geschützt im Verpackungseinsatz aufzunehmen.

Vorteilhaft ist es, wenn der Kunststoff Polyethylen (PE), insbesondere Polyethylen hoher Dichte (PE-HD), Polyurethan (PUR), Polysiloxan (Silikon) und/oder Polyethylenterephthalat (PET), insbesondere mit Glykol modifiziertes Polyethylenterephthalat (PETG), ist oder enthält. Mit derartigen Kunststoffen, insbesondere in geeigneter Dicke, kann beispielsweise eine Stabilität der Übergangsbereiche, also insbesondere eine Steifigkeit derselben, in der wie oben beschriebenen Weise eingestellt werden.

Günstig ist es, wenn das medizinische Objekt in Form eines Implantats, einer Implantatkomponente oder eines medizinischen Instruments ausgebildet ist. Der medizinische Verpackungseinsatz kann in Form und Größe insbesondere an das aufzunehmende Objekt angepasst sein.

Die eingangs gestellte Aufgabe wird bei einem medizinischen Verpackungssystem der eingangs beschriebenen Art erfindungsgemäß dadurch gelöst, dass der medizinische Verpackungseinsatz in Form eines der oben beschriebenen medizinischen Verpackungseinsätze ausgebildet ist.

Das medizinische Verpackungssystem weist dann die bereits oben in Verbindung mit bevorzugten Ausführungsformen von medizinischen Verpackungseinsätzen beschriebenen Vorteile auf. Wie erwähnt kann durch den Verpackungseinsatz insbesondere ein direkter Kontakt des mindestens einen medizinischen Objekts mit dem ersten Verpackungsbehälter verhindert werden. Dadurch kann es nicht zu einem Partikelabrieb aufgrund einer Relativbewegung zwischen dem mindestens einen medizinischen Objekt und dem ersten Verpackungsbehälter kommen.

Günstig ist es, wenn der erste Verpackungsbehälter ein wannenförmiges Aufnahmeteil und ein Verschlusselement umfasst, wenn das Aufnahmeteil den ersten Verpackungsbehälteraufnahmeraum definiert und wenn das Verschlusselement den ersten Verpackungsbehälteraufnahmeraum in einer Verschlussstellung verschließt, insbesondere keimdicht. Der erste Verpackungsbehälter kann insbesondere selbsttragend ausgebildet sein. Er kann jedoch auch biegeschlaff ausgebildet sein, beispielsweise in Form einer Tüte. Das Verschlusselement hat insbesondere den Vorteil, dass der Verpackungsbehälteraufnahmeraum zum Einsetzen oder Einführen eines Verpackungseinsatzes mit darin aufgenommenem medizinischen Objekt vollständig oder im Wesentlichen vollständig frei zugänglich ist. Verschlossen werden kann der Verpackungsbehälteraufnahmeraum mit dem Verschlusselement.

Einfach und sicher lässt sich der erste Verpackungsbehälter verschließen, wenn das Aufnahmeteil und das Verschlusselement in der Verschlussstellung durch Kleben und/oder Schweißen miteinander verbunden sind.

Eine Sterilverpackung kann durch den ersten Verpackungsbehälter auf einfache und kostengünstige Weise ausgebildet werden, wenn das Aufnahmeteil aus einer ersten Folie ausgebildet ist und wenn das Verschlusselement aus einer zweiten Folie ausgebildet ist. Das Aufnahmeteil und das Verschlusselement lassen sich bei dieser Ausgestaltung auf einfache Weise miteinander verbinden, insbesondere Kleben und/oder Verschweißen, wodurch eine definierte Sterilbarriere ausgebildet werden kann.

Um dem ersten Verpackungsbehälter insbesondere eine definierte Form zu verleihen, ist es günstig, wenn der erste Verpackungsbehälter durch thermisches Umformen ausgebildet ist und einen von einem den ersten Verpackungsbehälteraufnahmeraum umlaufenden Rand des ersten Verpackungsbehälters abstehenden und umlaufenden Verbindungsflansch aufweist und wenn der Verbindungsflansch eine Verbindungsflanschebene definiert. Beispielsweise kann so ein wannenförmiger erster Verpackungsbehälter ausgebildet werden. Der Verbindungsflansch ermöglicht insbesondere auf einfache Weise eine flächige Verbindung mit einem ebenen Verschlusselement, welches beispielsweise aus einem Schichtmaterial wie insbesondere einer Folie ausgebildet sein kann.

Um eine zuverlässige Sterilbarriere realisieren zu können, ist es günstig, wenn das Verschlusselement flächig mit dem Verbindungsflansch in der Verschlussstellung verbunden ist.

Ferner kann vorgesehen sein, dass das Verpackungssystem einen zweiten, insbesondere unsterilen, Verpackungsbehälter umfasst und dass der zweite Verpackungsbehälter einen zweiten Verpackungsbehälteraufnahmeraum zum Aufnehmen des ersten Verpackungsbehälters aufweist. Der zweite Verpackungsbehälter kann insbesondere eine Umverpackung für den ersten Verpackungsbehälter bilden. Beispielsweise kann dieser in Form eines Kartons ausgebildet sein, der den ersten Verpackungsbehälter beim Transport oder bei Lagerung vor Beschädigungen schützen kann.

Auf einfache und kostengünstige Weise lässt sich der zweite Verpackungsbehälter ausbilden, wenn er in Form einer Verpackungsschachtel ausgebildet ist. Insbesondere kann die Verpackungsschachtel aus Karton und/oder Pappe ausgebildet sein.

Vorzugsweise ist das Verpackungssystem in Form eines Sterilverpackungssystems zum sterilen Verpacken des mindestens einen medizinischen Objekts ausgebildet. Dies ermöglicht es insbesondere, medizinische Objekte, also beispielsweise Implantate, Implantatkomponenten oder medizinische Instrumente, nach ihrer Herstellung oder einer Wiederaufbereitung steril im ersten Verpackungsbehälter zu verpacken. Zum Schutz des ersten Verpackungsbehälters gegen unerwünschten Abrieb kann das mindestens eine medizinische Objekt vor dem Einbringen in den ersten Verpackungsbehälteraufnahmeraum in einen der oben beschriebenen Verpackungseinsätze eingebracht werden.

Günstig ist es, wenn das Verpackungssystem Gamma-sterilisierbar ausgebildet ist. Insbesondere können der Verpackungseinsatz und der erste Verpackungsbehälter Gamma-sterilisierbar ausgebildet sein. Durch diese Ausgestaltung, insbesondere durch Wahl geeigneter Materialien zur Ausbildung des ersten Verpackungsbehälters und des ersten Verpackungseinsatzes, kann das medizinische Objekt sogar noch dann sterilisiert werden, wenn es im ersten Verpackungsbehälteraufnahmeraum aufgenommen ist.

Ferner kann es vorteilhaft sein, wenn das Verpackungssystem einen Formkörper umfasst, welcher eine Aufnahme zum Aufnehmen des unverschlossenen Aufnahmeteils des ersten Verpackungsbehälters umfasst, wenn der Formkörper eine ebene Seitenfläche aufweist und wenn sich die Aufnahme ausgehend von der ebenen Seitenfläche in den Formkörper hinein erstreckt. Ein solcher Formkörper ermöglicht es insbesondere, den ersten Verpackungsbehälter, nämlich dessen wannenförmiges Aufnahmeteil, in die Aufnahme des Formkörpers einzuführen. Ein solcher Formkörper kann insbesondere einen ersten Verpackungsbehälter, welcher aus einem biegeschlaffen Material wie insbesondere einer Folie ausgebildet ist, zum Einsetzen des Verpackungseinsatzes stabilisieren.

Günstigerweise umgibt die Seitenfläche die Aufnahme allseitig. Dies ermöglicht es insbesondere, einen umlaufenden Verbindungsflansch des ersten Verpackungsbehälters über einen gesamten Umfang durch die Seitenfläche des Formkörpers zu stützen.

Die eingangs gestellte Aufgabe wird ferner bei einem Verfahren der eingangs beschriebenen Art erfindungsgemäß dadurch gelöst, dass der Verpackungseinsatz derart ausgerichtet wird, dass die zwei Laschen quer zur Schwerkraftrichtung ausgerichtet sind und sich die Behälterwanne entgegen der Schwerkraftrichtung erstreckt, dass die zwei Laschen gehalten oder auf eine Haltefläche aufgelegt werden und dass zum Umstülpen des Verpackungseinsatzes von der Grundstellung in die Verpackungsstellung mit dem medizinischen Objekt in Schwerkraftrichtung oder im Wesentlichen in Schwerkraftrichtung auf eine Außenfläche der Behälterwanne eine Umstülpkraft ausgeübt wird. Wenn sich die Behälterwanne entgegen der Schwerkraftrichtung erstreckt, ist sie zunächst in Schwerkraftrichtung weisend geöffnet. Ein Objekt kann in dieser Ausrichtung nur entgegen der Schwerkraftrichtung in die Behälterwanne eingeführt werden. Durch die beschriebene Ausrichtung ist es jedoch möglich, gleichzeitig mit dem Einführen des medizinischen Objekts in den Verpackungseinsatz den Verpackungseinsatz in der bereits mehrfach beschriebenen Weise umzustülpen, so dass die zwei in der Grundstellung voneinander weg weisenden Laschen nach Umstülpen des Verpackungseinsatzes aufeinander zu weisen und den Aufnahmeraum des umgestülpten Verpackungseinsatzes mindestens teilweise, insbesondere vollständig, verschließen. Die beschriebene Vorgehensweise ermöglicht es insbesondere, nur durch Kontakt zwischen dem vom Verpackungseinsatz aufzunehmenden medizinischen Objekt und dem Verpackungseinsatz den Verpackungseinsatz in der beschriebenen Weise umzustülpen. Dies erleichtert das Einbringen des Objekts in den Verpackungseinsatz. Insbesondere sind keine weiteren Hilfsmittel erforderlich, um die Laschen in eine Position zu bringen, in der sie den Aufnahmeraum verschließen. Dies ergibt sich durch das Umstülpen des Verpackungseinsatzes automatisch.

Günstig ist es, wenn ein Formkörper bereitgestellt wird, welcher eine Aufnahme zum Aufnehmen der Behälterwanne in der Verpackungsstellung umfasst und eine ebene Seitenfläche aufweist, wobei sich die Aufnahme ausgehend von der ebenen Seitenfläche in den Formkörper hinein erstreckt, und wenn der Verpackungseinsatz vor dem Verpacken des medizinischen Objekts in der Grundstellung derart auf den mit der Seitenfläche entgegen der Schwerkraftrichtung weisend ausgerichteten Formkörper aufgelegt wird, dass die zwei Laschen auf der Seitenfläche des Formkörpers diesseits und jenseits der Aufnahme aufliegen und sich die Behälterwanne vom Formkörper entgegen der Schwerkraftrichtung weg erstreckt. Mit anderen Worten wölbt sich die Behälterwanne des Verpackungseinsatzes direkt über der Aufnahme des Formkörpers von diesem weg weisend. Wird ein medizinisches Objekt mit der so ausgerichteten Behälterwanne in Kontakt gebracht und eine Umstülpkraft ausgeübt, beispielsweise aufgrund der auf das medizinische Objekt wirkenden Gewichtskraft, kann beim Bewegen des medizinischen Objekts in Richtung auf die Aufnahme hin und in diese hinein der Verpackungseinsatz in der beschriebenen Weise umgestülpt werden, so dass die in der Grund- oder Ausgangsstellung voneinander weg weisenden Laschen in der Verpackungsstellung, wenn die Behälterwanne in die Aufnahme hinein umgestülpt ist, den Aufnahmeraum des Verpackungseinsatzes verschließen.

Vorteilhaft ist es, wenn vor dem Auflegen des Verpackungseinsatzes ein erster Verpackungsbehälters, welcher ein wannenförmiges, einen ersten Verpackungsbehälteraufnahmeraum definierendes Aufnahmeteil umfasst und einen von einem den ersten Verpackungsbehälteraufnahmeraum begrenzenden umlaufenden Rand des ersten Verpackungsbehälters abstehenden umlaufenden Verbindungsflansch aufweist, derart auf den Formkörper gelegt wird, dass das wannenförmige Aufnahmeteil in die Aufnahme des Formkörpers eingreift und der Verbindungsflansch auf der Seitenfläche des Formkörpers aufliegt. Diese Vorgehensweise ermöglicht es insbesondere, den ersten Verpackungsbehälter am Formkörper so zu positionieren, dass der erste Verpackungsbehälteraufnahmeraum frei zugänglich ist. Der Verbindungsflansch liegt wie beschrieben allseitig auf der Seitenfläche des Formkörpers auf. In einem nächsten Schritt kann wie oben erläutert der Verpackungseinsatz mit entgegen der Schwerkraftrichtung gewölbter Behälterwanne auf den Formkörper aufgelegt werden. Der Verbindungsflansch liegt dann mindestens teilweise zwischen der Seitenfläche des Formkörpers und den Laschen des Verpackungseinsatzes. Wird wie oben erläutert ein medizinisches Objekt in den Verpackungseinsatz eingeführt durch gleichzeitiges Umstülpen des Verpackungseinsatzes, ist das medizinische Objekt nach dem vollständigen Umstülpen des Verpackungseinsatzes vollständig in diesem aufgenommen. Der Verpackungseinsatz wiederum befindet sich vollständig aufgenommen im wannenförmigen Aufnahmeteil des ersten Verpackungsbehälters. Optional kann in einem nächsten Schritt das wannenförmige Aufnahmeteil des ersten Verpackungsbehälters mit einem Verschlusselement, beispielsweise einer Oberfolie, verschlossen werden durch Verkleben und/oder Verschweißen. In einem optionalen weiteren Schritt kann das so steril verpackte medizinische Objekt in einen zweiten Verpackungsbehälter eingebracht werden. Der zweite Verpackungsbehälter kann insbesondere eine Umverpackung bilden, um den sterilen ersten Verpackungsbehälter für Transport und Lagerung zu schützen.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Ansicht eines Verpackungseinsatzes;
- Figur 2:: eine Schnittansicht längs Linie 2-2 in Figur 1;
- Figur 3:: eine Ansicht des Verpackungseinsatz aus Figur 1 von unten in Richtung des Pfeils A;
- Figur 4:: eine Seitenansicht des Verpackungseinsatzes aus Figur 3 in Richtung des Pfeils B;
- Figur 5:: eine schematische Darstellung eines medizinischen Verpackungssystems beim Einführen eines medizinischen Objekts in einen Verpackungseinsatz;
- Figur 6:: eine Ansicht ähnlich Figur 5 mit teilweise in den Verpackungseinsatz eingeführtem medizinischen Objekt;
- Figur 7:: eine Ansicht ähnlich Figur 6 mit noch weiter in den Verpackungseinsatz eingeführtem medizinischen Objekt;
- Figur 8:: eine teilweise durchbrochene Draufsicht auf die Anordnung in Figur 9 in Richtung des Pfeils C;
- Figur 9:: eine Schnittansicht längs Linie 9-9 in Figur 8;
- Figur 10:: eine schematische Schnittansicht eines weiteren Ausführungsbeispiels eines medizinischen Verpackungssystems;
- Figur 11:: eine Draufsicht auf ein weiteres Ausführungsbeispiel eines medizinischen Verpackungssystems aus Figur 12 in Richtung des Pfeils D; und
- Figur 12:: eine Schnittansicht längs Linie 12-12 in Figur 11.

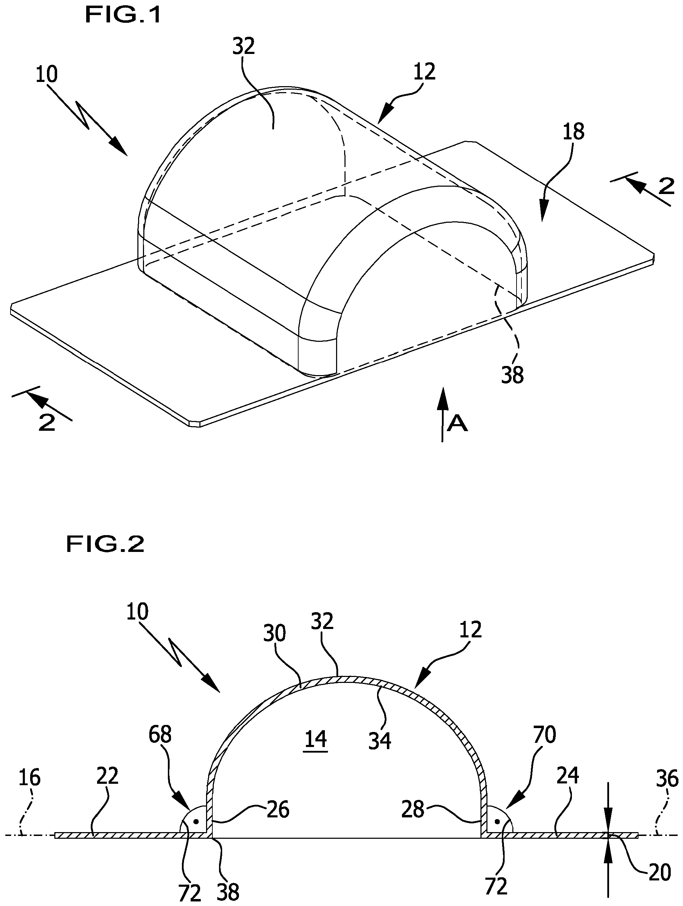

In Figur 1 ist schematisch ein Ausführungsbeispiel eines Verpackungseinsatzes für ein medizinisches Verpackungssystem 76 zum Aufnehmen mindestens eines medizinischen Objekts 78 dargestellt und mit dem Bezugszeichen 10 bezeichnet.

Der Verpackungseinsatz 10 umfasst eine Behälterwanne 12, die einen Aufnahmeraum 14 definiert.

Der Verpackungseinsatz 10 ist durch thermisches Umformen aus einem ebenen, eine Schichtmaterialebene 16 definierenden Schichtmaterial 18 ausgebildet, das eine Dicke 20 aufweist.

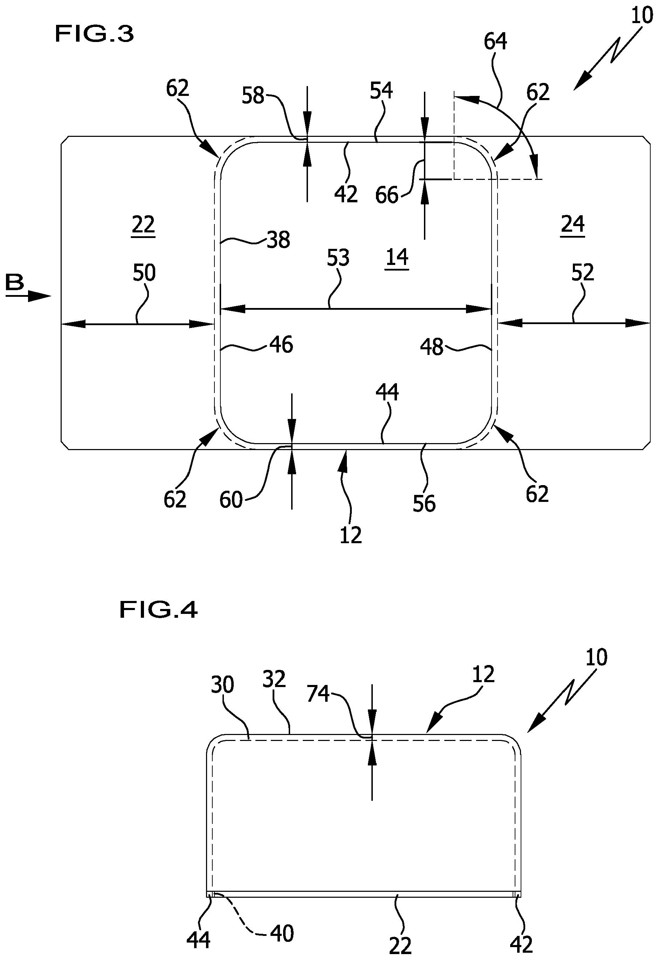

An der Behälterwanne 12 sind zwei voneinander weg weisende Laschen 22 und 24 ausgebildet, die in einer in den Figuren 1 bis 4 dargestellten Grundstellung quer von aufeinander zu weisenden Wandflächen 26 und 28 der Behälterwanne 12 abstehen.

Die Behälterwanne 12 ist von einer Behälterwannenwand 30 begrenzt. In der in den Figuren 1 bis 4 schematisch dargestellten Grundstellung des Verpackungseinsatzes 10 definiert die Behälterwannenwand 30 eine äußere Behälterwandfläche 32 und eine innere Behälterwandfläche 34. Die innere Behälterwandfläche 34 begrenzt in der Grundstellung den Aufnahmeraum 14. Die äu-βere Behälterwannenfläche 32 weist in der Grundstellung von der Behälterwanne 12 weg.

Die zwei Laschen 22 und 24 sind eben ausgebildet. Sie definieren in der Grundstellung eine gemeinsame Laschenebene 36. Diese ist durch die Schichtmaterialebene 16 definiert.

Die Behälterwanne 12 weist einen umlaufenden Rand 38 auf, welcher in der Schichtmaterialebene 16 liegt und von dem sich die zwei Laschen 22 und 24 weg erstrecken. Die Behälterwanne 12 definiert ferner eine Öffnung 40, die vom Rand 38 begrenzt wird und die in der Schichtmaterialebene 16 liegt.

Die Öffnung 40 ist im Wesentlichen rechteckig ausgebildet und wird begrenzt von zwei Längsseiten 42 und 44, die parallel zueinander verlaufen, und zwei senkrecht zu den Längsseiten 42 und 44 verlaufenden, sich parallel zueinander erstreckenden Querseiten 46 und 48.

Die zwei Laschen 22 und 24 sind jeweils an einer Querseite 46 beziehungsweise 48 des Rands 38 angeordnet beziehungsweise ausgebildet.

Die zwei Laschen 22 und 24 weisen jeweils eine Länge 50 beziehungsweise 52 ausgehend von der Behälterwanne 12 weg auf. Die Längen 50 und 52 entsprechen mindestens etwa einem halben Abstand 53 zwischen den parallel zueinander verlaufenden Querseiten 46 und 48.

Die Längen 50 und 52 der Laschen 22 und 24 sind jedoch nicht länger als der Abstand 53.

In der beschriebenen Weise bilden die Laschen 22 und 24 jeweils einen flanschartigen Abschnitt an der Behälterwanne 12 aus.

Die Längsseiten 42 und 44 des Rands 38 definieren Randabschnitte 54 beziehungsweise 56, die flanschlos ausgebildet sind. Dies bedeutet insbesondere, dass die Randabschnitte 54 und 56 jeweils eine Dicke 58 beziehungsweise 60 aufweisen, die bei dem in den Figuren dargestellten Ausführungsbeispiel der Dicke 20 des Schichtmaterials 18 entspricht. Die Dicken 58 und 60 können jedoch auch etwas von der Dicke 20 abweichen, was sich aufgrund des thermischen Umformens des Schichtmaterials 18 ergeben kann.

Die Längsseiten 42 und 44 und die Querseiten 46 und 48 sind über einen Eckbereich 62 verbunden. Jeder der insgesamt vier Eckbereiche 62, die bei dem in den Figuren dargestellten Ausführungsbeispiel des Verpackungseinsatzes 10 vorgesehen sind, verbinden eine der beiden Längsseiten 42 und 44 mit einer der beiden Querseiten 46 und 48.

Die Eckbereiche 62 bilden jeweils einen Abschnitt des Rands 38. Jeder Eckbereich 62 verläuft gekrümmt, und zwar konvex gekrümmt vom Aufnahmeraum 14 weg weisend. In der Grundstellung ist dadurch auch die äußere Behälterwandfläche 32, die sich ausgehend von den Laschen 22 und 24 vom Rand 38 weg erstreckt, von der Behälterwanne 12 weg weisend konvex gekrümmt.

Jeder Eckbereich 62 erstreckt sich in der Schichtmaterialebene 16. Ferner erstreckt sich jeder Eckbereich 62 über einen Umfangswinkel 64 in einem Bereich von etwa 85° bis etwa 95°. Bei dem in den Figuren dargestellten Ausführungsbeispiel weist der Umfangswinkel 64 einen Wert von etwa 90° auf.

Ein Krümmungsradius 66 der Eckbereiche 62 weist einen Wert in einem Bereich von etwa 3 mm bis etwa 20 mm auf.

Bei dem in den Figuren dargestellten Ausführungsbeispiel erstrecken sich die Querseiten 46 und 48 des Rands 38 zwischen den sich an diese anschließenden Eckbereichen 62 geradlinig. Auch die Längsseiten 42 und 44 des Rands 38 erstrecken sich jeweils zwischen zwei Eckbereichen 62 geradlinig beziehungsweise im Wesentlichen geradlinig.

Bei einem alternativen Ausführungsbeispiel sind die Längsseiten 42 und 44 des Rands 38 zwischen den zwei Eckbereichen 62 vom Verpackungseinsatz 10 weg weisend konvex gekrümmt.

Zwischen der Behälterwanne 12 und jeweils einer der beiden Laschen 22 und 24 ist ein Übergangsbereich 68 beziehungsweise 70 ausgebildet. Zwischen der Behälterwanne 12 und der jeweiligen Lasche 22 beziehungsweise 24 ist ein Flanschwinkel 72 eingeschlossen. Der Flanschwinkel 72 weist bei dem in den Figuren dargestellten Ausführungsbeispiel einen Wert in einem Bereich von etwa 85° bis etwa 95° auf, nämlich etwa 90°.

Das beschriebene Ausführungsbeispiel des Verpackungseinsatzes 10 ist einstückig ausgebildet, nämlich monolithisch. Eine Wandstärke 74 des Verpackungseinsatzes 10 liegt bei dem beschriebenen Ausführungsbeispiel in einem Bereich von etwa 0,05 mm bis etwa 10 mm. Bei dem beschriebenen Ausführungsbeispiel entspricht die Wandstärke 74 der Dicke 20 des Schichtmaterials 18.

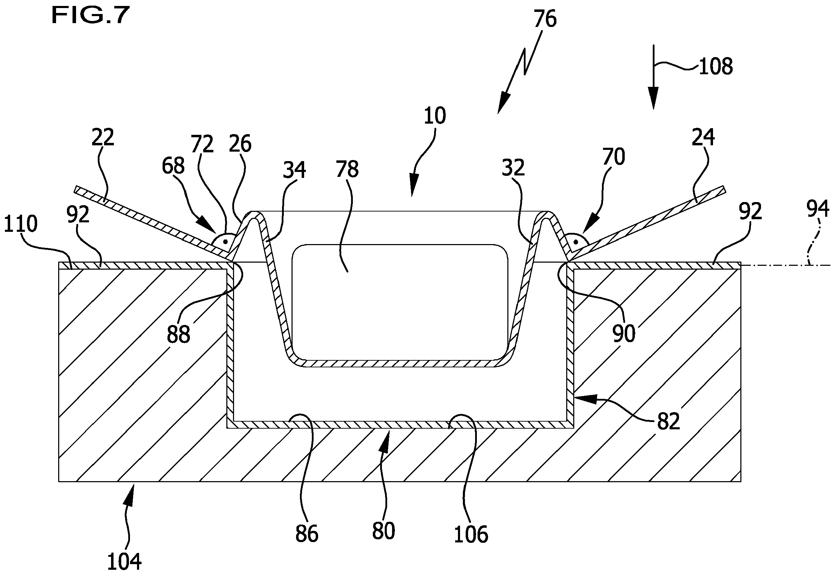

Der Verpackungseinsatz 10 ist umfasst von einem in den Figuren 7 bis 9 schematisch dargestellten medizinischen Verpackungssystem 76. Dieses dient dem Verpacken eines oder mehrerer medizinischer Objekte 78. In den Figuren 7 bis 9 ist ein solches medizinisches Objekt 78 schematisch dargestellt.

Bei dem medizinischen Objekt 78 kann es sich insbesondere um ein Implantat, eine Implantatkomponente oder ein medizinisches Instrument handeln.

Das Verpackungssystem 76 umfasst einen ersten Verpackungsbehälter 80.

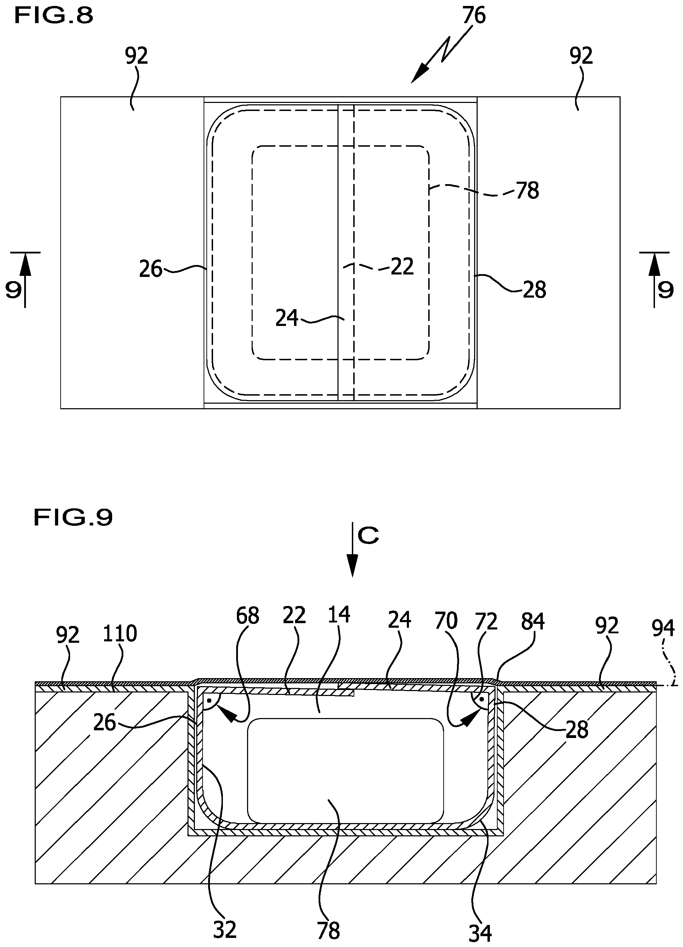

Der erste Verpackungsbehälter 80 umfasst ein wannenförmiges Aufnahmeteil 82 und ein in Figur 9 schematisch dargestelltes Verschlusselement 84.

Das Aufnahmeteil 82 definiert einen ersten Verpackungsbehälteraufnahmeraum 86, der eine Öffnung 88 definiert. Das in den Figuren 8 und 9 schematisch dargestellte Verschlusselement 84 verschließt die Öffnung 88 des Aufnahmeteils 82 und damit den ersten Verpackungsbehälteraufnahmeraum 86 in einer schematisch in Figur 9 dargestellten Verschlussstellung. In der Verschlussstellung ist der erste Verpackungsbehälter 80 keimdicht verschlossen.

Der erste Verpackungsbehälteraufnahmeraum 86 dient zum Aufnehmen eines oder mehrerer medizinischer Verpackungseinsätze 10, in denen jeweils ein oder mehrere medizinische Objekte 78 aufgenommen sind.

Der erste Verpackungsbehälter 80 ist durch thermisches Umformen aus einem ersten Verpackungsbehältermaterial ausgebildet. Der erste Verpackungsbehälter 80 weist einen den ersten Verpackungsbehälteraufnahmeraum 86 umgebenden umlaufenden Rand 90 auf. Vom Rand 90 weg erstreckt sich ein umlaufender Verbindungsflansch 92, welcher die Öffnung 88 vollumfänglich umgibt. Der Verbindungsflansch 92 definiert eine Verbindungsflanschebene 94.

In der schematisch in Figur 9 dargestellten Verschlussstellung des ersten Verpackungsbehälters 80 ist das Verschlusselement 84 flächig mit dem Verbindungsflansch 92 verbunden. Diese Verbindung ist bei dem in den Figuren 8 und 9 dargestellten Ausführungsbeispiel des ersten Verpackungsbehälters 80 durch Kleben und/oder Schweißen realisiert.

Das Aufnahmeteil 82 ist bei dem beschriebenen Ausführungsbeispiel des ersten Verpackungsbehälters 80 aus einer ersten Folie, also aus einem biegeschlaffen Material, ausgebildet. Das Verschlusselement 84 ist aus einer zweiten Folie ausgebildet, also ebenfalls aus einem biegeschlaffen Material.

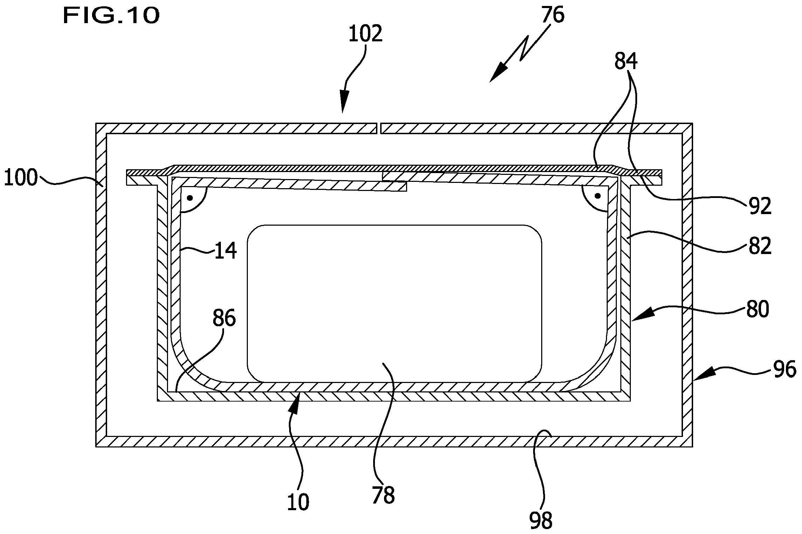

Figur 10 zeigt beispielhaft ein weiteres Ausführungsbeispiel eines Verpackungssystems 76. Dieses dient ebenfalls zum Verpacken eines medizinischen Objekts 78. Das medizinische Objekt 78 ist im Verpackungseinsatz 10 aufgenommen. Der Verpackungseinsatz 10 wiederum ist im ersten Verpackungsbehälter 80 aufgenommen.

Das in Figur 10 schematisch dargestellte Verpackungssystem 76 umfasst ferner einen zweiten Verpackungsbehälter 96.

Der zweite Verpackungsbehälter 96 definiert einen zweiten Verpackungsbehälteraufnahmeraum 98 zum Aufnehmen des ersten Verpackungsbehälters 80.

Bei dem in Figur 10 dargestellten Ausführungsbeispiel ist der zweite Verpackungsbehälter 96 in Form einer Verpackungsschachtel 100 aus Karton beziehungsweise Pappe ausgebildet.

Die beschriebenen Ausführungsbeispiele von Verpackungssystemen 76 sind in Form von Sterilverpackungssystemen 102 zum sterilen Verpacken eines oder mehrerer medizinischer Objekte 78 ausgebildet. Eine Sterilverpackung dieser Sterilverpackungssysteme 102 wird durch den ersten Verpackungsbehälter 80 gebildet.

Optional umfasst das Verpackungssystem 10 einen in den Figuren 7 und 9 schematisch dargestellten Formkörper 104. Dieser dient in einer nachfolgend noch näher beschriebenen Weise als Verpackungs- beziehungsweise Einführhilfe zum Einführen eines oder mehrerer Objekte 78 in den Verpackungseinsatz 10 und in den ersten Verpackungsbehälteraufnahmeraum 86 des Aufnahmeteils 82 des ersten Verpackungsbehälters 80.

Der Formkörper 104 umfasst eine Aufnahme 106 in Form einer Ausnehmung am im Wesentlichen quaderförmig ausgebildeten Formkörper 104. Von einer entgegen der durch einen Pfeil 108 symbolisierten Schwerkraftrichtung weisenden ebenen Seitenfläche erstreckt sich die Aufnahme 106 in den Formkörper 104 hinein.

Der erste Verpackungsbehälter 80, insbesondere das Aufnahmeteil 82 sowie die Aufnahme 106 des Formkörpers 104 sind in Form und Größe aneinander angepasst derart, dass der unverschlossene Aufnahmeteil 82 in der Aufnahme 106 aufgenommen werden kann, wie dies schematisch in den Figuren 7 und 9 dargestellt ist. Der Aufnahmeteil 82 kleidet sozusagen die Aufnahme 106 aus. Der Verbindungsflansch 92 liegt flächig auf der Seitenfläche 110 auf.

Damit der Verbindungsflansch 92 allseitig durch den Formkörper 104 unterstützt werden kann, umgibt die Seitenfläche 110 die Aufnahme 106 allseitig.

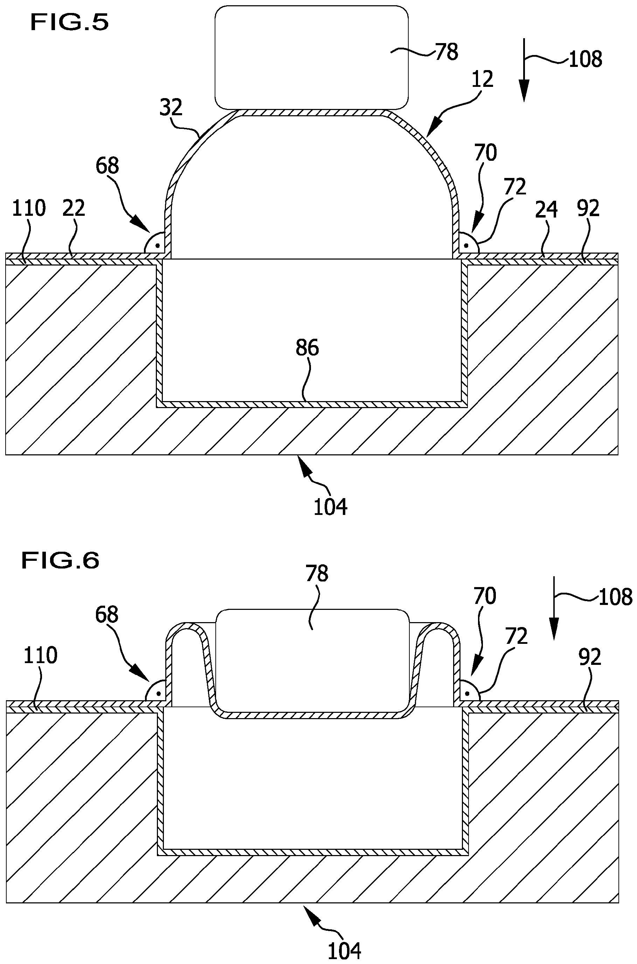

Die Funktionsweise des Verpackungssystems 76 wird nachfolgend in Verbindung mit den Figuren 5 bis 9 näher erläutert.

Zum Verpacken eines medizinischen Objekts 78 im ersten Verpackungsbehälter 80 wird zunächst die Aufnahme 106 in der beschriebenen Weise mit dem Aufnahmeteil 82 des ersten Verpackungsbehälters 80 ausgekleidet. Der Verbindungsflansch 92 liegt flächig auf der Seitenfläche 110 auf, welche somit den aus einem biegeschlaffen Material gebildeten Aufnahmeteil 82 unterstützt.

Wie schematisch in Figur 5 dargestellt wird dann der Verpackungseinsatz 10 auf den Formkörper 104 aufgelegt, und zwar derart, dass sich die Behälterwanne 12 von der Aufnahme 106 weg weisend wölbt. Die beiden Laschen 22 und 24 liegen diesseits und jenseits der Aufnahme 106 sowie des ersten Verpackungsbehälteraufnahmeraums 86 auf dem Verbindungsflansch auf.

In Figur 5 ist schematisch dargestellt, wie das medizinische Objekt 78 mit der äußeren Behälterwandfläche 32 des die Grundstellung einnehmenden Verpackungseinsatzes 10 in Kontakt tritt.

Durch Wirkung der Schwerkraft auf das Objekt 78 bewegt sich dieses in Richtung auf die Aufnahme 106 hin. Dabei verformt das Objekt 78 die Behälterwanne 12 und stülpt diese um, wie dies schematisch in den Figuren 6 und 7 dargestellt ist. Die Übergangsbereiche 68 und 70 weisen eine Steifigkeit derart auf, dass der Flanschwinkel 72 während und nach dem Überführen des Verpackungseinsatzes 10 von der Grundstellung in die schematisch in den Figuren 8 und 9 dargestellte Verpackungsstellung erhalten oder im Wesentlichen erhalten bleibt.

Beim Umstülpen oder Umschlagen der Behälterwanne 12 ändert sich eine Orientierung der Wandflächen 26 und 28. Wie beschrieben weisen diese in der Grundstellung aufeinander zu. Beim Umstülpen des umstülpbar ausgebildeten Verpackungseinsatzes 10 werden diese jeweils um 180° gewendet, so dass sie in der Verpackungsstellung wie schematisch in Figur 9 dargestellt voneinander weg weisen.

Aufgrund der Steifigkeit der Übergangsbereiche 68 und 70 klappen auch die Laschen 22 und 24 um 180° um und weisen in der Verpackungsstellung aufeinander zu. Durch die Steifigkeit der Übergangsbereiche 68 und 70 werden also die Laschen 22 und 24 in der beschriebenen Weise umgeklappt und verschlie-βen die umgestülpte Behälterwanne 12, bei der nun die in der Grundstellung äußere Wandfläche 32 praktisch eine innere Wandfläche der Behälterwanne 12 bildet. Der nun von der Behälterwanne 12 definierte Aufnahmeraum 14 wird in der Verschlussstellung also durch die äußere Behälterwandfläche 32 begrenzt.

In der beschriebenen Weise kann der Verpackungseinsatz 10, nämlich der von diesem in der Verschlussstellung definierte Aufnahmeraum 14, automatisch verschlossen werden beim Umstülpen des Verpackungseinsatzes 10 in der beschriebenen Weise. Ein manuelles Angreifen am Verpackungseinsatz, um die Laschen 22 und 24 in eine Stellung zu bringen, in der sie mindestens teilweise überlappen und den Aufnahmeraum 14 ganz oder teilweise verschließen, ist nicht erforderlich.

Das Aufnahmeteil 82 kann in einem nächsten Schritt mit dem Verschlusselement 84 verschlossen werden. Hierzu wird das Verschlusselement 84 wie schematisch in Figur 9 dargestellt auf den Flansch und den Verpackungseinsatz 10 übergreifend aufgelegt. Der Verbindungsflansch 92 und das Verschlusselement 84 werden verklebt und/oder verschweißt.

Wenn der Flanschwinkel 72 im Übergangsbereich 68 beziehungsweise 70 beim Umstülpen der Behälterwanne 12 erhalten bleibt, ergibt sich eine Differenz zwischen dem Flanschwinkel 72 in der Grundstellung und dem Flanschwinkel 72 in der Verpackungsstellung von 0°. Mithin ist also die angegebene Differenz nicht größer als 30°.

Der wie beschrieben verschlossene erste Verpackungsbehälter 80 mit darin aufgenommenem Verpackungseinsatz 10 und in diesem aufgenommenen Objekt 78 kann dann wie beschrieben in einen zweiten Verpackungsbehälter 96 eingebracht werden, beispielsweise zum Transport oder zur Lagerung.

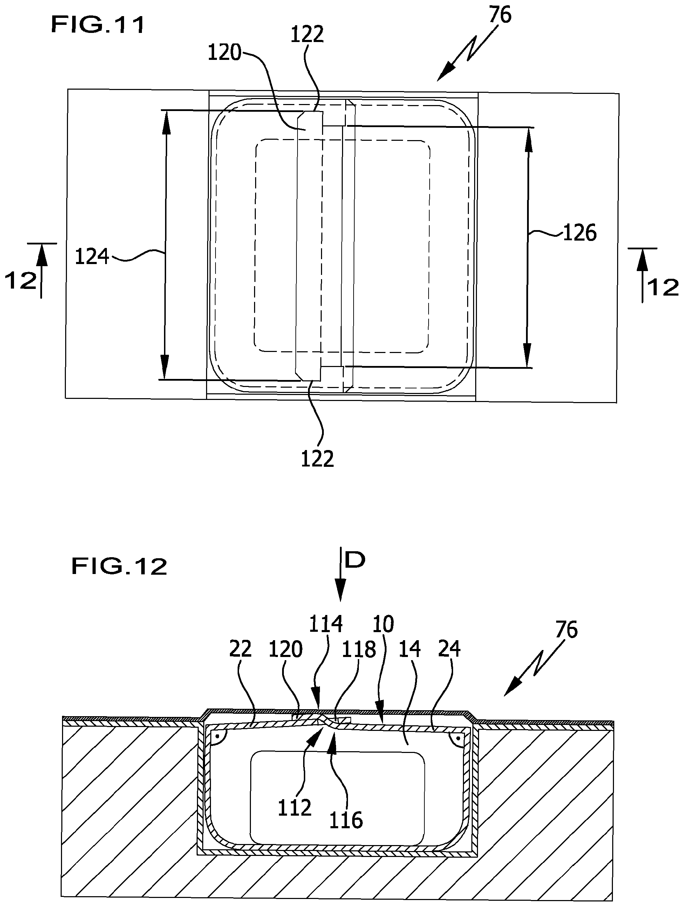

In den Figuren 11 und 12 ist schematisch ein weiteres Ausführungsbeispiel eines Verpackungssystems 76 dargestellt. Es unterscheidet sich von dem oben beschriebenen Verpackungssystem 76 lediglich durch die Ausgestaltung des Verpackungseinsatzes 10.

Anders als bei dem oben beschriebenen Verpackungseinsatz 10 umfasst der Verpackungseinsatz 10 des in den Figuren 11 und 12 dargestellten Ausführungsbeispiels zusätzlich eine Verschlusseinrichtung 112.

Die Verschlusseinrichtung 112 umfasst miteinander zusammenwirkende erste und zweite Verschlusselemente 114 und 116. Das zweite Verschlusselement 116 ist in Form eines Schlitzes 118 an der Lasche 22 ausgebildet und erstreckt sich parallel zu den Querseiten 46 und 48. Das erste Verschlusselement 114 ist in Form eines Verschlussvorsprungs 120 ausgebildet, der ein freies Ende der Lasche 24 definiert. Der Verschlussvorsprung 120 weist parallel zu den Querseiten 46 und 48 voneinander weg weisend abstehende Rückhaltevorsprünge 122 auf. Ein Abstand 124 voneinander weg weisender Endflächen der Rückhaltevorsprünge 122 ist größer als eine Länge 126 des Schlitzes 118 parallel zu den Querseiten 46 und 48. Ist der Verschlussvorsprung 120 durch den Schlitz 118 hindurchgeführt, verhindern die Rückhaltevorsprünge 122 ein Zurückziehen des Verschlussvorsprungs 120. Die beiden Laschen 22 und 24 stehen in der Verschlussstellung dann kraft- und/oder formschlüssig miteinander in Eingriff und halten den Aufnahmeraum 14 verschlossen.

Die oben beschriebenen Ausführungsbeispiele von Verpackungseinsätzen 10 können insbesondere aus einem Kunststoff ausgebildet sein, beispielsweise einem thermoplastischen Kunststoff. Es kann sich dabei um einen geschäumten oder extrudierten Kunststoff handeln, beispielsweise geschäumtes Polyethylen oder geschäumtes Polyethylen hoher Dichte (PE-HD).

Alternative Ausführungsbeispiele von Verpackungseinsätzen 10 sind aus Kunststoffen gebildet, die Polyurethan (PUR), Polysiloxan (Silikon) und/oder Polyethylenterephthalat (PET), insbesondere mit Glykol modifiziertes Polyethylenterephthalat (PETG), sind oder enthalten.

Die beschriebenen Ausführungsbeispiele von Verpackungseinsätzen 10 verhindern aufgrund ihrer oben beschriebenen Ausgestaltung in der Verpackungsstellung einen direkten Kontakt zwischen dem verpackten medizinischen Objekt 78 und dem ersten Verpackungsbehälter 80. So kann es nicht zu einem Abrieb am ersten Verpackungsbehälter 80 kommen und damit nicht zu einer Kontamination der medizinischen Objekte 78 mit Abriebpartikeln.

### Bezugszeichenliste

- 10: Verpackungseinsatz
- 12: Behälterwanne
- 14: Aufnahmeraum
- 16: Schichtmaterialebene
- 18: Schichtmaterial
- 20: Dicke
- 22: Lasche
- 24: Lasche
- 26: Wandfläche
- 28: Wandfläche
- 30: Behälterwannenwand
- 32: äußere Behälterwandfläche
- 34: innere Behälterwandfläche
- 36: Laschenebene
- 38: Rand
- 40: Öffnung
- 42: Längsseite
- 44: Längsseite
- 46: Querseite
- 48: Querseite
- 50: Länge
- 52: Länge
- 53: Abstand
- 54: Randabschnitt
- 56: Randabschnitt
- 58: Dicke
- 60: Dicke
- 62: Eckbereich
- 64: Umfangswinkel
- 66: Krümmungsradius
- 68: Übergangsbereich
- 70: Übergangsbereich
- 72: Flanschwinkel
- 74: Wandstärke
- 76: Verpackungssystem
- 78: Objekt
- 80: erster Verpackungsbehälter
- 82: Aufnahmeteil
- 84: Verschlusselement
- 86: erster Verpackungsbehälteraufnahmeraum
- 88: Öffnung
- 90: Rand
- 92: Verbindungsflansch
- 94: Verbindungsflanschebene
- 96: zweiter Verpackungsbehälter
- 98: zweiter Verpackungsbehälteraufnahmeraum
- 100: Verpackungsschachtel
- 102: Sterilverpackungssystem
- 104: Formkörper
- 106: Aufnahme
- 108: Pfeil
- 110: Seitenfläche
- 112: Verschlusseinrichtung
- 114: erstes Verschlusselement
- 116: zweites Verschlusselement
- 118: Schlitz
- 120: Verschlussvorsprung
- 122: Rückhaltevorsprung
- 124: Abstand
- 126: Länge

## Patentansprüche

1. Medizinischer Verpackungseinsatz (10) für ein medizinisches Verpackungssystem (76) zum Aufnehmen mindestens eines medizinischen Objekts (78), wobei der Verpackungseinsatz (10) eine Behälterwanne (12) umfasst, die einen Aufnahmeraum (14) definiert, und durch thermisches Umformen aus einem ebenen, eine Schichtmaterialebene (16) definierenden Schichtmaterial (18) ausgebildet ist, wobei an der Behälterwanne (12) zwei voneinander weg weisende Laschen (22, 24) ausgebildet sind, die in einer Grundstellung quer, insbesondere senkrecht, von aufeinander zu weisenden Wandflächen (26, 28) der Behälterwanne (12) abstehen, **dadurch gekennzeichnet, dass** der Verpackungseinsatz (10) umstülpbar ausgebildet ist und durch Umstülpen von der Grundstellung in eine Verpackungsstellung, in der die in der Grundstellung aufeinander zu weisenden Wandflächen (26, 28) voneinander weg weisen und die zwei Laschen (22, 24) aufeinander zu weisen und den Aufnahmeraum (14) mindestens teilweise, insbesondere vollständig, verschließen, überführbar ist.

2. Medizinischer Verpackungseinsatz nach Anspruch 1, **dadurch gekennzeichnet, dass**
a) die Behälterwanne (12) von einer Behälterwannenwand (30) begrenzt ist, dass die Behälterwannenwand (30) in der Grundstellung des Verpackungseinsatzes (10) eine äußere Behälterwandfläche (32) und eine innere Behälterwandfläche (34) definiert, dass die innere Behälterwandfläche (32) in der Grundstellung den Aufnahmeraum (14) begrenzt und dass in der Verpackungsstellung die äußere Behälterwandfläche (34) den Aufnahmeraum (14) begrenzt
und/oder
b) die zwei Laschen (22, 24) mindestens eines der folgenden:
- eben oder im Wesentlichen eben ausgebildet sind und in der Grundstellung und/oder in der Verpackungsstellung eine gemeinsame Laschenebene (36) definieren,
wobei insbesondere die Laschenebene (36) durch die Schichtmaterialebene (16) definiert ist;
- in der Verpackungsstellung einander mindestens teilweise, insbesondere vollständig, überlappen.

3. Medizinischer Verpackungseinsatz nach einem der voranstehenden Ansprüche, **dadurch gekennzeichnet, dass** die Behälterwanne (12) einen umlaufenden Rand (38) aufweist, dass der Rand (38) in der Schichtmaterialebene (16) liegt und dass die zwei Laschen (22, 24) sich vom Rand (38) weg erstrecken.

4. Medizinischer Verpackungseinsatz nach Anspruch 3, **dadurch gekennzeichnet, dass** der Rand (38) zwei Längsseiten (42, 44) und zwei Querseiten (46, 48) aufweist und dass die zwei Laschen (22, 24) jeweils an einer Querseite (46, 48) des Rands (38) angeordnet oder ausgebildet sind.

5. Medizinischer Verpackungseinsatz nach Anspruch 4, **dadurch gekennzeichnet, dass**
a) die zwei Laschen (22, 24) eine Länge (50, 52) ausgehend von der Behälterwanne (12) weg aufweisen, die mindestens etwa einem halben Abstand (53) zwischen den zwei Querseiten (46, 48) entspricht,
wobei insbesondere die Länge (50, 52) jeder Lasche (22, 24) maximal etwa einem Abstand (53) zwischen den zwei Querseiten (46, 48) entspricht,
und/oder
b) die Längsseiten (42, 44) des Rands (38) definierende Randabschnitte (54, 56) flanschlos ausgebildet sind.

6. Medizinischer Verpackungseinsatz nach Anspruch 4 oder 5, **dadurch gekennzeichnet, dass** die Längsseiten (42, 44) und die Querseiten (46, 48) des Rands (38) über einen Eckbereich (62) verbunden sind, dass der Eckbereich (62) einen Abschnitt des Rands (38) bildet und dass der Eckbereich (62) gekrümmt verläuft,
wobei insbesondere
a) sich der Eckbereich (62) in der Schichtmaterialebene (16) und über einen Umfangswinkel (64) in einem Bereich von etwa 85° bis etwa 95°, insbesondere etwa 90°, erstreckt
und/oder
b) ein Krümmungsradius (66) des Eckbereichs (62) einen Wert in einem Bereich von etwa 3 mm bis etwa 20 mm aufweist, insbesondere etwa 4 mm bis etwa 8 mm,
und/oder
c) sich die Querseiten (46, 48) des Rands (38) zwischen zwei Eckbereichen (62) geradlinig erstrecken
und/oder
d) sich die Längsseiten (42, 44) des Rands (38) zwischen zwei Eckbereichen (62) geradlinig oder im Wesentlichen geradlinig erstrecken oder dass sich die Längsseiten (42, 44) des Rands (38) zwischen zwei Eckbereichen (62) konvex vom Verpackungseinsatz (10) weg weisend erstrecken.

7. Medizinischer Verpackungseinsatz nach einem der voranstehenden Ansprüche, **dadurch gekennzeichnet, dass** Übergangsbereiche (68, 70) zwischen der Behälterwanne (12) und den zwei Laschen (22, 24) eine Steifigkeit aufweisen derart, dass ein zwischen der Behälterwanne (12) und der jeweiligen Lasche (22, 24) in den Übergangsbereichen (68, 70) eingeschlossener Flanschwinkel (72) nach dem Überführen des Verpackungseinsatzes (10) von der Grundstellung in die Verpackungsstellung erhalten bleibt oder im Wesentlichen erhalten bleibt,
wobei insbesondere
a) der Flanschwinkel (72) einen Wert in einem Bereich von etwa 85° bis etwa 95° aufweist, insbesondere etwa 90°,
und/oder
b) eine Differenz des Flanschwinkels (72) in der Grundstellung und des Flanschwinkels (72) in der Verpackungsstellung nicht größer als 30° ist, insbesondere nicht größer als 15°.

8. Medizinischer Verpackungseinsatz nach einem der voranstehenden Ansprüche, **dadurch gekennzeichnet, dass**
a) der Verpackungseinsatz (10) einstückig, insbesondere monolithisch, ausgebildet ist
und/oder
b) eine Wandstärke (74) des Verpackungseinsatzes (10) in einem Bereich von etwa 0,05 mm bis etwa 10 mm liegt,
wobei insbesondere die Wandstärke (74) einer Dicke (20) des Schichtmaterials (18) entspricht oder im Wesentlichen entspricht.

9. Medizinischer Verpackungseinsatz nach einem der voranstehenden Ansprüche, **dadurch gekennzeichnet, dass** der Verpackungseinsatz (10) aus einem Kunststoff, insbesondere aus einem thermoplastischen Kunststoff, ausgebildet ist,
wobei der Kunststoff
a) geschäumt oder extrudiert oder in Form eines Vliesstoffs ausgebildet ist
und/oder
b) Polyethylen (PE), insbesondere Polyethylen hoher Dichte (PE-HD), Polyurethan (PUR), Polysiloxan (Silikon) und/oder Polyethylenterephthalat (PET), insbesondere mit Glykol modifiziertes Polyethylenterephthalat (PETG) ist oder enthält.

10. Medizinisches Verpackungssystem (76) zum, insbesondere sterilen, Verpacken mindestens eines medizinischen Objekts (78), wobei das Verpackungssystem (76) einen ersten Verpackungsbehälter (80) und mindestens einen medizinischen Verpackungseinsatz (10) umfasst, wobei der erste Verpackungsbehälter (80) einen ersten Verpackungsbehälteraufnahmeraum (86) definiert zum Aufnehmen des mindestens einen medizinischen Verpackungseinsatzes (10) und des mindestens einen medizinischen Objekts (78), das im medizinischen Verpackungseinsatz (10) aufgenommen ist, **dadurch gekennzeichnet, dass** der medizinische Verpackungseinsatz (10) in Form eines medizinischen Verpackungseinsatzes (10) nach einem der voranstehenden Ansprüche ausgebildet ist.

11. Medizinisches Verpackungssystem nach Anspruch 10, **dadurch gekennzeichnet, dass** der erste Verpackungsbehälter (80) ein wannenförmiges Aufnahmeteil (82) und ein Verschlusselement (84) umfasst, dass das Aufnahmeteil (82) den ersten Verpackungsbehälteraufnahmeraum (86) definiert und dass das Verschlusselement (84) den ersten Verpackungsbehälteraufnahmeraum (86) in einer Verschlussstellung verschließt, insbesondere keimdicht,
wobei insbesondere das Aufnahmeteil (82)
a) und das Verschlusselement (84) in der Verschlussstellung durch Kleben und/oder Schweißen miteinander verbunden sind und/oder
b) aus einer ersten Folie ausgebildet ist und dass das Verschlusselement (84) aus einer zweiten Folie ausgebildet ist.

12. Medizinisches Verpackungssystem nach Anspruch 10 oder 11, **dadurch gekennzeichnet, dass**
a) der erste Verpackungsbehälter (80) durch thermisches Umformen ausgebildet ist und einen von einem den ersten Verpackungsbehälteraufnahmeraum (86) umlaufenden Rand (38) des ersten Verpackungsbehälters (80) abstehenden und umlaufenden Verbindungsflansch (92) aufweist und dass der Verbindungsflansch (92) eine Verbindungsflanschebene (94) definiert,
wobei insbesondere das Verschlusselement (84) flächig mit dem Verbindungsflansch (92) in der Verschlussstellung verbunden ist, und/oder
b) das Verpackungssystem (76) mindestens eines der folgenden:
- einen zweiten, insbesondere unsterilen, Verpackungsbehälter (96) umfasst und dass der zweite Verpackungsbehälter (96) einen zweiten Verpackungsbehälteraufnahmeraum (98) zum Aufnehmen des ersten Verpackungsbehälters (80) aufweist, wobei insbesondere der zweite Verpackungsbehälter (96) in Form einer Verpackungsschachtel (100) ausgebildet ist, insbesondere aus Karton und/oder Pappe;
- in Form eines Sterilverpackungssystem (102) zum sterilen Verpacken des mindestens einen medizinischen Objekts (78) ausgebildet ist;
- Gamma-sterilisierbar ausgebildet ist, insbesondere der Verpackungseinsatz (10) und der erste Verpackungsbehälter (80).

13. Medizinisches Verpackungssystem nach Anspruch 11 oder 12, **dadurch gekennzeichnet, dass** das Verpackungssystem (76) einen Formkörper (104) umfasst, welcher eine Aufnahme (106) zum Aufnehmen des unverschlossenen Aufnahmeteils (82) des ersten Verpackungsbehälters (80) umfasst, dass der Formkörper (104) eine ebene Seitenfläche (110) aufweist und dass sich die Aufnahme (106) ausgehend von der ebenen Seitenfläche (110) in den Formkörper (104) hinein erstreckt,
wobei insbesondere die Seitenfläche (110) die Aufnahme (106) allseitig umgibt.

14. Verfahren zum Verpacken eines medizinischen Objekts (78) in einem Verpackungseinsatz (10) nach einem der Ansprüche 1 bis 9, **dadurch gekennzeichnet, dass** der Verpackungseinsatz (10) derart ausgerichtet wird, dass die zwei Laschen (22, 24) quer zur Schwerkraftrichtung (108) ausgerichtet sind und sich die Behälterwanne (12) entgegen der Schwerkraftrichtung (108) erstreckt, dass die zwei Laschen (22, 24) gehalten oder auf eine Haltefläche (110) aufgelegt werden und dass zum Umstülpen des Verpackungseinsatzes (10) von der Grundstellung in die Verpackungsstellung mit dem medizinischen Objekt (78) in Schwerkraftrichtung (108) oder im Wesentlichen in Schwerkraftrichtung (108) auf eine Außenfläche (32) der Behälterwanne (12) eine Umstülpkraft ausgeübt wird.

15. Verfahren nach Anspruch 14, **dadurch gekennzeichnet, dass** ein Formkörper (104) bereitgestellt wird, welcher eine Aufnahme (106) zum Aufnehmen der Behälterwanne (12) in der Verpackungsstellung umfasst und eine ebene Seitenfläche (110) aufweist, wobei sich die Aufnahme (106) ausgehend von der ebenen Seitenfläche (110) in den Formkörper (104) hinein erstreckt, und dass der Verpackungseinsatz (10) vor dem Verpacken des medizinischen Objekts (78) in der Grundstellung derart auf den mit der Seitenfläche (110) entgegen der Schwerkraftrichtung (108) weisend ausgerichteten Formkörper (104) aufgelegt wird, dass die zwei Laschen (22, 24) auf der Seitenfläche (110) des Formkörpers (104) diesseits und jenseits der Aufnahme (106) aufliegen und sich die Behälterwanne (12) vom Formkörper (104) entgegen der Schwerkraftrichtung (108) erstreckt,
wobei insbesondere vor dem Auflegen des Verpackungseinsatzes (10) ein erster Verpackungsbehälters (80), welcher ein wannenförmiges, einen ersten Verpackungsbehälteraufnahmeraum (86) definierendes Aufnahmeteil (82) umfasst und einen von einem den ersten Verpackungsbehälteraufnahmeraum (86) begrenzenden umlaufenden Rand (90) des ersten Verpackungsbehälters (80) abstehenden umlaufenden Verbindungsflansch (92) aufweist, derart auf den Formkörper (104) gelegt wird, dass das wannenförmige Aufnahmeteil (82) in die Aufnahme (106) des Formkörpers (104) eingreift und der Verbindungsflansch (92) auf der Seitenfläche (110) des Formkörpers (104) aufliegt.

## Claims

1. Medical packaging insert (10) for a medical packaging system (76) for accommodating at least one medical object (78), wherein the packaging insert (10) comprises a container trough (12), which defines a receiving space (14), and is formed from a planar layer material (18) defining a layer material plane (16) by thermal reshaping, wherein formed on the container trough (12) are two tabs (22, 24) pointing away from one another, which in a basic position project transversely, in particular perpendicularly, from wall faces (26, 28) of the container trough (12) facing toward one another, **characterized in that** the packaging insert (10) is configured to be evertable and is transferable by eversion from the basic position into a packaging position in which the wall faces (26, 28) facing toward one another in the basic position face away from one another and the two tabs (22, 24) point toward one another and at least partially, in particular completely, close the receiving space (14).

2. Medical packaging insert in accordance with Claim 1, **characterized in that**
a) the container trough (12) is delimited by a container trough wall (30), **in that** the container trough wall (30) in the basic position of the packaging insert (10) defines an outer container wall face (32) and an inner container wall face (34), **in that** the inner container wall face (32) in the basic position delimits the receiving space (14), and **in that** in the packaging position the outer container wall face (34) delimits the receiving space (14),
and/or
b) the two tabs (22, 24) at least one of the following:
- are of planar or substantially planar configuration and in the basic position and/or in the packaging position define a common tab plane (36),
wherein, in particular, the tab plane (36) is defined by the layer material plane (16);
- in the packaging position at least partially, in particular completely, overlap one another.

3. Medical packaging insert in accordance with any one of the preceding Claims, **characterized in that** the container trough (12) has a peripheral rim (38), **in that** the rim (38) is located in the layer material plane (16), and **in that** the two tabs (22, 24) extend away from the rim (38).

4. Medical packaging insert in accordance with Claim 3, **characterized in that** the rim (38) has two longitudinal sides (42, 44) and two transverse sides (46, 48) and **in that** the two tabs (22, 24) are each arranged or formed on a transverse side (46, 48) of the rim (38).

5. Medical packaging insert in accordance with Claim 4, **characterized in that**
a) the two tabs (22, 24) have a length (50, 52) commencing away from the container trough (12), which corresponds to at least about half a distance (53) between the two transverse sides (46, 48), wherein, in particular, the length (50, 52) of each tab (22, 24) corresponds to at most about a distance (53) between the two transverse sides (46, 48),
and/or
b) rim portions (54, 56) defining the longitudinal sides (42, 44) of the rim (38) are of flangeless configuration.

6. Medical packaging insert in accordance with Claim 4 or 5, **characterized in that** the longitudinal sides (42, 44) and the transverse sides (46, 48) of the rim (38) are connected by way of a corner region (62), **in that** the corner region (62) forms a portion of the rim (38), and **in that** the corner region (62) extends in a curved manner,
wherein, in particular,
a) the corner region (62) extends in the layer material plane (16) and over a circumferential angle (64) in a range of about 85° to about 95°, in particular about 90°,
and/or
b) a radius of curvature (66) of the corner region (62) has a value in a range of about 3 mm to about 20 mm, in particular about 4 mm to about 8 mm,
and/or
c) the transverse sides (46, 48) of the rim (38) extend rectilinearly between two corner regions (62)
and/or
d) the longitudinal sides (42, 44) of the rim (38) extend rectilinearly or substantially rectilinearly between two corner regions (62) or **in that** the longitudinal sides (42, 44) of the rim (38) extend between two corner regions (62) pointing convexly away from the packaging insert (10).

7. Medical packaging insert in accordance with any one of the preceding Claims, **characterized in that** transition regions (68, 70) between the container trough (12) and the two tabs (22, 24) have a stiffness such that a flange angle (72) enclosed between the container trough (12) and the respective tab (22, 24) in the transition regions (68, 70) is maintained or substantially maintained after transferring the packaging insert (10) from the basic position into the packaging position,
wherein, in particular,
a) the flange angle (72) has a value in a range of about 85° to about 95°, in particular about 90°,
and/or
b) a difference of the flange angle (72) in the basic position and the flange angle (72) in the packaging position is not greater than 30°, in particular not greater than 15°.

8. Medical packaging insert in accordance with any one of the preceding Claims, **characterized in that**
a) the packaging insert (10) is of one piece, in particular monolithic, configuration,
and/or
b) a wall thickness (74) of the packaging insert (10) is in a range of about 0.05 mm to about 10 mm,
wherein, in particular, the wall thickness (74) corresponds or substantially corresponds to a thickness (20) of the layer material (18).

9. Medical packaging insert in accordance with any one of the preceding Claims, **characterized in that** the packaging insert (10) is made of a plastic, in particular of a thermoplastic material,
wherein the plastic
a) is foamed or extruded or is configured in the form of a non-woven fabric
and/or
b) is or contains polyethylene (PE), in particular high density polyethylene (PE-HD), polyurethane (PUR), polysiloxane (silicone), and/or polyethylene terephthalate (PET), in particular polyethylene terephthalate modified with glycol (PETG).

10. Medical packaging system (76) for, in particular sterile, packaging of at least one medical object (78), wherein the packaging system (76) comprises a first packaging container (80) and at least one medical packaging insert (10), wherein the first packaging container (80) defines a first packaging container receiving space (86) for accommodating the at least one medical packaging insert (10) and the at least one medical object (78) that is accommodated in the medical packaging insert (10), **characterized in that** the medical packaging insert (10) is configured in the form of a medical packaging insert (10) in accordance with any one of the preceding Claims.

11. Medical packaging system in accordance with Claim 10, **characterized in that** the first packaging container (80) comprises a trough-shaped receiving part (82) and a closure element (84), **in that** the receiving part (82) defines the first packaging container receiving space (86), and **in that** the closure element (84) closes, in particular in a germ-tight manner, the first packaging container receiving space (86) in a closed position,
wherein, in particular, the receiving part (82)
a) and the closure element (84) in the closed position are connected to one another by adhesion and/or welding,
and/or
b) is made from a first film and **in that** the closure element (84) is made from a second film.

12. Medical packaging system in accordance with Claim 10 or 11, **characterized in that**
a) the first packaging container (80) is formed by thermal reshaping and has a peripheral connecting flange (92) projecting from a rim (38) of the first packaging container (80) surrounding the first packaging container receiving space (86), and **in that** the connecting flange (92) defines a connecting flange plane (94),
wherein, in particular, the closure element (84) is connected in surface-to-surface contact with the connecting flange (92) in the closed position,
and/or
b) the packaging system (76) at least one of the following:
- comprises a second, in particular unsterile, packaging container (96) and **in that** the second packaging container (96) has a second packaging container receiving space (98) for accommodating the first packaging container (80),
wherein, in particular, the second packaging container (96) is configured in the form of a packaging box (100), in particular made of cardboard and/or paperboard;
- is configured in the form of a sterile packaging system (102) for sterile packaging of the at least one medical object (78);
- is configured to be able to be gamma-sterilized, in particular the packaging insert (10) and the first packaging container (80).

13. Medical packaging system in accordance with Claim 11 or 12, **characterized in that** the packaging system (76) comprises a mold (104), which comprises a receptacle (106) for accommodating the unclosed receiving part (82) of the first packaging container (80), **in that** the mold (104) has a planar side face (110), and **in that** the receptacle (106) extends commencing from the planar side face (110) into the mold (104), wherein, in particular, the side face (110) surrounds the receptacle (106) on all sides.

14. Method for packaging a medical object (78) in a packaging insert (10) in accordance with any one of Claims 1 to 9, **characterized in that** the packaging insert (10) is oriented in such a way that the two tabs (22, 24) are oriented transversely to the direction of gravity (108) and the container trough (12) extends counter to the direction of gravity (108), **in that** the two tabs (22, 24) are held or are placed on a main face (110), and **in that** for everting the packaging insert (10) from the basic position into the packaging position with the medical object (78) an eversion force is exerted in the direction of gravity (108) or substantially in the direction of gravity (108) on an outer face (32) of the container trough (12).

15. Method in accordance with Claim 14, **characterized in that** a mold (104) is provided, which has a receptacle (106) for accommodating the container trough (12) in the packaging position and has a planar side face (110), wherein the receptacle (106) extends from the planar side face (110) into the mold (104), and **in that** the packaging insert (10) before packaging the medical object (78) in the basic position is placed on the mold (104) oriented facing with the side face (110) counter to the direction of gravity (104) in such a way that the two tabs (22, 24) rest on the side face (110) of the mold (104) on both sides of the receptacle (106) and the container trough (12) extends from the mold (104) counter to the direction of gravity (108),
wherein, in particular, before placing the packaging insert (10) a first packaging container (80), which comprises a trough-shaped receiving part (82) defining a first packaging container receiving space (86) and has a peripheral connecting flange (92) projecting from a peripheral rim (90) of the first packaging container (80) delimiting the first packaging container receiving space (86), is placed on the mold (104) in such a way that the trough-shaped receiving part (82) engages into the receptacle (106) of the mold (104) and the connecting flange (92) rests on the side face (110) of the mold (104).

## Revendications

1. Insert d'emballage médical (10) pour un système d'emballage médical (76) pour recevoir au moins un objet médical (78), où l'insert d'emballage (10) comprend une cuvette de récipient (12), qui définit un espace de réception (14), et est formé par formage thermique à partir d'un matériau en couche (18) plat définissant un plan de matériau en feuille (16), où deux languettes (22, 24) s'écartant l'une de l'autre sont formées sur la cuvette de récipient (12), qui, dans une position de base, s'écartent
transversalement, en particulier perpendiculairement, de surfaces de paroi (26, 28) de la cuvette de récipient (12) tournées l'une vers l'autre, **caractérisé en ce que** l'insert d'emballage (10) est conçu pour pouvoir être retourné et peut être amené, par retournement, de la position de base à une position d'emballage dans laquelle les surfaces de paroi (26, 28) qui, dans la position de base, sont tournées l'une vers l'autre, sont tournées à l'opposé l'une de l'autre et les deux languettes (22, 24) sont tournées l'une vers l'autre et ferment au moins en partie, en particulier totalement, l'espace de réception (14).

2. Insert d'emballage médical selon la revendication 1, **caractérisé en ce que**
a) la cuvette de récipient (12) est limitée par une paroi de cuvette de récipient (30), **en ce que** la paroi de cuvette du récipient (30) définit dans la position de base de l'insert d'emballage (10) une surface de paroi de récipient extérieure (32) et une surface de paroi de récipient intérieure (34), **en ce que** la surface de paroi de récipient intérieure (32) limite l'espace de réception (14) dans la position de base et **en ce que** la surface de paroi de récipient extérieure (34) limite l'espace de réception (14) dans la position d'emballage
et/ou
b) les deux languettes (22, 24) comportent au moins l'un des suivants:
- sont conçues plates ou sensiblement plates et dans la position de base et/ou dans la position d'emballage définissent un plan de languette commun (36),
où en particulier le plan de languette (36) est défini par le plan de matériau en couche (16);
- se chevauchent au moins en partie, en particulier totalement, dans la position d'emballage.

3. Insert d'emballage médical selon l'une des revendications précédentes, **caractérisé en ce que** la cuvette de récipient (12) présente un bord périphérique (38), **en ce que** le bord (38) est situé dans le plan de matériau en couche (16) et **en ce que** les deux languettes (22, 24) s'étendent en s'éloignant du bord (38).

4. Insert d'emballage médical selon la revendication 3, **caractérisé en ce que** le bord (38) présente deux côtés longitudinaux (42, 44) et deux côtés transversaux (46, 48) et **en ce que** les deux languettes (22, 24) sont disposées ou formées chacune sur un côté transversal (46, 48) du bord (38).

5. Insert d'emballage médical selon la revendication 4, **caractérisé en ce que**
a) les deux languettes (22, 24) présentent une longueur (50, 52) en partant de la cuvette de récipient (12) qui correspond au moins sensiblement à la moitié de la distance (53) entre les deux côtés transversaux (46, 48), où en particulier la longueur (50, 52) de chaque languette (22, 24) correspond au maximum à sensiblement une distance (53) entre les deux côtés transversaux (46, 48),
et/ou
b) les sections de bord (54, 56) définies par les côtés longitudinaux (42, 44) du bord (38) sont conçues sans bride.

6. Insert d'emballage médical selon la revendication 4 ou 5, **caractérisé en ce que** les côtés longitudinaux (42, 44) et les côtés transversaux (46, 48) du bord (38) sont reliés par une zone d'angle (62), **en ce que** la zone d'angle (62) forme une section du bord (38) et **en ce que** la zone d'angle (62) s'étend de manière incurvée,
où en particulier
a) la zone d'angle (62) s'étend dans le plan de matériau en couche (16) et sur un angle circonférentiel (64) dans une plage d'environ 85° à environ 95°, en particulier environ 90°,
et/ou
b) un rayon de courbure (66) de la zone d'angle (62) présente une valeur dans une plage d'environ 3 mm à environ 20 mm, en particulier d'environ 4 mm à environ 8 mm,
et/ou
c) les côtés transversaux (46, 48) du bord (38) s'étendent en ligne droite entre deux zones d'angle (62)
et/ou
d) les côtés longitudinaux (42, 44) du bord (38) s'étendent en ligne droite ou sensiblement en ligne droite entre deux zones d'angle (62) ou **en ce que** les côtés longitudinaux (42, 44) du bord (38) s'étendent entre deux zones d'angle (62) de manière convexe en s'écartant de l'insert d'emballage (10).

7. Insert d'emballage médical selon l'une des revendications précédentes, **caractérisé en ce que** des zones de transition (68, 70) entre la cuvette de récipient (12) et les deux languettes (22, 24) présentent une rigidité de telle sorte qu'un angle de bride (72) inclus entre la cuvette de récipient (12) et la languette (22, 24) respective dans les zones de transition (68, 70) est conservé ou sensiblement conservé après le passage de l'insert d'emballage (10) de la position de base à la position d'emballage, où en particulier
a) l'angle de bride (72) présente une valeur dans une plage d'environ 85° à environ 95°, en particulier environ 90°,
et/ou
b) une différence de l'angle de bride (72) dans la position de base et de l'angle de bride (72) dans la position d'emballage n'est pas supérieure à 30°, en particulier pas supérieure à 15°.

8. Insert d'emballage médical selon l'une des revendications précédentes, **caractérisé en ce que**
a) l'insert d'emballage (10) est formé en une seule pièce, en particulier monolithique,
et/ou
b) une épaisseur de paroi (74) de l'insert d'emballage (10) est située dans une plage d'environ 0,05 mm à environ 10 mm, où en particulier l'épaisseur de paroi (74) correspond ou correspond sensiblement à une épaisseur (20) du matériau en couche (18).

9. Insert d'emballage médical selon l'une des revendications précédentes, **caractérisé en ce que** l'insert d'emballage (10) est formé en une matière synthétique, en particulier en une matière synthétique thermoplastique,
où la matière synthétique
a) est expansée ou extrudée ou sous la forme d'un non-tissé
et/ou
b) est ou contient du polyéthylène (PE), en particulier du polyéthylène haute densité (PE-HD), du polyuréthane (PUR), du polysiloxane (silicone) et/ou du polyéthylène téréphtalate (PET), en particulier du polyéthylène téréphtalate modifié avec du glycol (PETG).

10. Système d'emballage médical (76) pour l'emballage, en particulier stérile, d'au moins un objet médical (78), où le système d'emballage (76) comprend un premier récipient d'emballage (80) et au moins un insert d'emballage médical (10), où le premier récipient d'emballage (80) définit un premier espace de réception de récipient d'emballage (86) pour recevoir le au moins un insert d'emballage médical (10) et le au moins un objet médical (78), qui est reçu dans l'insert d'emballage médical (10), **caractérisé en ce que** l'insert d'emballage médical (10) est conçu sous la forme d'un insert d'emballage médical (10) selon l'une quelconque des revendications précédentes.

11. Système d'emballage médical selon la revendication 10, **caractérisé en ce que** le premier récipient d'emballage (80) comprend une partie de réception en forme de cuvette (82) et un élément de fermeture (84), **en ce que** la partie de réception (82) définit le premier espace de réception de récipient d'emballage (86) et **en ce que** l'élément de fermeture (84) ferme le premier espace de réception de récipient d'emballage (86) dans une position de fermeture, en particulier de manière étanche aux germes,
où en particulier la partie de réception (82)
a) et l'élément de fermeture (84) sont reliés l'un à l'autre par collage et/ou soudage dans la position de fermeture
et/ou
b) est formée à partir d'un premier film et **en ce que** l'élément de fermeture (84) est formé à partir d'un second film.

12. Système d'emballage médical selon la revendication 10 ou 11, **caractérisé en ce que**
a) le premier récipient d'emballage (80) est formé par thermoformage et comporte une bride de liaison (92) s'étendant depuis un bord (38) entourant le premier espace de réception de récipient d'emballage (86) du premier récipient d'emballage (80) et périphérique et **en ce que** la bride de liaison (92) définit un plan de bride de liaison (94), où en particulier l'élément de fermeture (84) est relié avec la bride de liaison (92) à plat dans la position de fermeture,
et/ou
b) le système d'emballage (76) comprend au moins l'un des éléments suivants:
- un deuxième récipient d'emballage (96), en particulier non stérile, et **en ce que** le deuxième récipient d'emballage (96) comporte un deuxième espace de réception de récipient d'emballage (98) pour recevoir le premier récipient d'emballage (80),
où en particulier le deuxième récipient d'emballage (96) est conçu sous forme d'une boîte d'emballage (100), en particulier en carton et/ou en carton d'emballage,
- est conçu sous la forme d'un système d'emballage stérile (102) pour l'emballage stérile du au moins un objet médical (78);
- est conçu stérilisable aux rayons gamma, en particulier l'insert d'emballage (10) et le premier récipient d'emballage (80).

13. Système d'emballage médical selon la revendication 11 ou 12, **caractérisé en ce que** le système d'emballage (76) comprend un corps façonné (104) qui comprend un réceptacle (106) pour recevoir la partie de réception non fermée (82) du premier récipient d'emballage (80), **en ce que** le corps façonné (104) présente une surface latérale plane (110) et **en ce que** le réceptacle (106) s'étend dans le corps façonné (104) en partant de la surface latérale plane (110),
où en particulier la surface latérale (110) entoure le réceptacle (106) de tous les côtés.

14. Procédé pour emballer un objet médical (78) dans un insert d'emballage (10) selon l'une quelconque des revendications 1 à 9, **caractérisé en ce que** l'insert d'emballage (10) est orienté de telle manière que les deux languettes (22, 24) sont orientées transversalement à la direction de la gravité (108) et la cuvette de récipient (12) s'étend à l'encontre de la direction de la gravité (108), **en ce que** les deux languettes (22, 24) sont maintenues ou mises sur une surface de maintien (110) et **en ce que**, pour le retournement de l'insert d'emballage (10) de la position de base dans la position d'emballage avec l'objet médical (78), une force de retournement est exercée dans la direction de la gravité (108) ou sensiblement dans la direction de la gravité (108) sur une surface extérieure (32) de la cuvette de récipient (12).

15. Procédé selon la revendication 14, **caractérisé en ce qu'**un corps façonné (104) est fourni, qui comprend un réceptacle (106) pour recevoir la cuvette de récipient (12) dans la position d'emballage et présente une surface latérale plane (110), où le réceptacle (106) en partant de la surface latérale plane (110) s'étend dans le corps façonné (104), et **en ce que** l'insert d'emballage (10) avant l'emballage de l'objet médical (78) est placé dans la position de base sur le corps façonné (104) orienté avec la surface latérale (110) contre la direction de la gravité (108), de telle sorte que les deux languettes (22, 24) reposent sur la surface latérale (110) du corps façonné (104) de part et d'autre du réceptacle (106) et la cuvette de récipient (12) s'étend depuis le corps façonné (104) contre la direction de la gravité (108), où en particulier avant la mise en place de l'insert d'emballage (10), un premier récipient d'emballage (80) qui comprend une partie de réception (82) en forme de cuvette, définissant un premier espace de réception de récipient d'emballage (86) et présente une bride de liaison périphérique (92) partant d'un bord périphérique (90) limitant le premier espace de réception de récipient d'emballage (86) du premier récipient d'emballage (80), est mis en place sur le corps façonné (104) de telle sorte que la partie de réception en forme de cuvette (82) pénètre dans le réceptacle (106) du corps façonné (104) et la bride de liaison (92) repose sur la surface latérale (110) du corps façonné (104).
